(19) Europäisches Patentamt
European Patent Office
Office européen des brevets

(11) Numéro de publication: **0 357 470 B1**

# (12) FASCICULE DE BREVET EUROPEEN

(45) Date de publication de fascicule du brevet: **30.03.94**

(51) Int. Cl.[5]: **C07C 209/50**, C07C 211/08, B01J 23/86

(21) Numéro de dépôt: **89401930.6**

(22) Date de dépôt: **05.07.89**

Jointe à la demande no. 89908212.7/0377732 (numéro de dépôt/numéro de publication de la demande européenne) par décision du 29.08.91.

## (54) Procédé d'obtention de N,N-diméthyl - N-alkylamines.

(30) Priorité: **08.07.88 FR 8809280**

(43) Date de publication de la demande:
**07.03.90 Bulletin 90/10**

(45) Mention de la délivrance du brevet:
**30.03.94 Bulletin 94/13**

(84) Etats contractants désignés:
**AT BE CH DE ES FR GB GR IT LI LU NL SE**

(56) Documents cités:
**EP-A- 0 144 467**
**DE-C- 712 098**
**FR-A- 1 408 714**
**US-A- 3 190 922**
**US-A- 3 444 204**

**JOURNAL OF APPLIED CHEMISTRY OF THE USSR, vol. 53, no. 8, partie 2, 10 fevrier 1981; L. P. PASHKOVA et al, "Hydrogenation of N,N-dimethylamides of fatty acids to the corresponding dimethylalkylamines"**

**PATENT ABSTRACTS OF JAPAN, vol. 6, no. 219 (C-132)(1097), 2 novembre 1982; & JP-A-57 123 127**

(73) Titulaire: **CECA S.A.**
**22, Place des Vosges**
**F-92400 Courbevoie(FR)**

(72) Inventeur: **Brouard, René**
**87, rue Saint Lazare**
**F-95290 l'Isle-Adam(FR)**
Inventeur: **Forquy, Christian**
**Ouartier COOS**
**F-64360 Monein(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 357 470 B1

## Description

La présente invention se rapporte à un procédé perfectionné pour l'obtention de N,N-diméthyl-N-alkylamines à longue chaîne par hydrogénation catalytique des diméthylamides correspondants.

Il s'agit d'obtenir par cette voie des N,N-diméthyl-N-alkylamines exemptes d'alcools lourds destinées, entre autres, à la fabrication de sels d'ammonium quaternaire pour la détergence ou de bactéricides ou désinfectants industriels.

TECHNIQUE ANTERIEURE

On sait produire des N,N-diméthyl-N-alkylamines par réaction de la diméthylamine sur un halogénure d'alkyle, sur un alcol gras ou sur une alpha-oléfine. Ce sont des voies industrielles, mais qui font appel à des matières premières onéreuses.

Une voie plus économique consiste à faire réagir la diméthylamine sur un acide gras pour former le N,N-diméthyl-alkylamide, puis à hydrogéner le N,N-diméthylalkylamide gras en N,N-diméthyl-N-alkylamine. On en connaît plusieurs modes de réalisation industrielle, qui opèrent par hydrogénation catalytique sous pression d'hydrogène; par exemple :

- le procédé divulgué dans le brevet US N° 3 190 922 déposé par la Société General Mills, opère sur catalyseur au chromite de cuivre, dont il est dit incidemment qu'il peut être stabiisé avec de l'oxyde de baryum, en présence de diméthylamine et sous flux circulant d'hydrogène; mais les catalyseurs utilisés, qu'ils soient ou non stabilisés au baryum, s'avèrent perdre une trop grande part de leur activité et de leur sélectivité;
- le procédé selon le brevet US N° 3 444 204, procédé en continu basé sur l'utilisation de catalyseurs du même type, en lit fixe sous forte pression (250 bars) ;
- le procédé décrit par L. Pashkova et m. Yakushkin dans J. of Applied Chem. of USSR, 53, 8, pp. 1398-1401 (1980), qui se distingue du précédent essentiellement en ce qu'on opère à pression atmosphérique;
- le brevet US N° 4 448 998 de la Société Procter et Gamble, qui représente une tentative pour améliorer la qualité des produits finis bruts, avec l'utilisation d'un catalyseur d'hydrogénation au chromite de cuivre associé à une zéolite en proportion non catalytique (18% par rapport à la charge), en vue de capter l'eau produite au cours de l'hydrogénation de l'amide et de minimiser les réactions parasites qu'elle engendre.

Ces procédés connus de l'art antérieur présentent en commun les inconvénients de ne pas fournir des N,N-diméthyl-N-alkylamines grasses de la qualité souhaitée pour au moins deux raisons.

- La pureté du produit final est insuffisante, et en particulier la teneur en alcools gras est trop élevée. Or ces alcools sont d'autant plus difficilement séparables par distillation qu'ils bouent très près des N,N-diméthyl-N-alkylamines et qu'il s'agit non pas de séparer un alcool gras de la N,N-diméthyl-N-alkylamine homologue, mais de séparer un ensemble d'alcools gras d'un ensemble de N,N-diméthyl-N-alkylamines dont la répartition des chaînes est celles des acides gras des huiles de départ, à savoir huiles de coco, de palmiste, etc... On s'en rendra aisément compte en consultant le tableau ci-dessous des températures comparées d'ébullition sous 20 mm de mercure (2,7 kPa) entre les N,N-diméthylalkylamines et les n-alcanols :

| longueur de chaîne alkyle | 8C | 10C | 12C | 14C | 16C | 18C |
|---|---|---|---|---|---|---|
| NN diméthyl alkylamine | 71 | 112 | 140 | 169 | 191 | 215 |
| alcanol | 101 | 126 | 150 | 177 | 198 | 220 |

- Le recyclage du catalyseur au chromite de cuivre, opération économiquement nécessaire, entraîne une diminution de son activité et accuse sa non-spécificité, ce qui se solde à chaque cycle par une augmentation de la teneur en alcools.

EXPOSE DE L'INVENTION

Il a été maintenant trouvé que l'utilisation d'un catalyseur au chromite de cuivre dopé au manganèse permettait de façon tout à fait imprévue de remédier aux inconvénients signalés. La présente invention réside dans un procédé d'obtention de N,N-diméthyl-N-alkylamines de formule générale

$$R-N<\begin{matrix}CH_3\\CH_3\end{matrix}$$

où R est un reste alkyle ou alkényle comprenant de 12 à 24 atomes de carbone, et préférentiellement d'amines à chaîne alkyle en $C_{12}$-$C_{14}$ , contenant moins de 1% d'alcools gras, par hydrogénation catalytique des N,N-diméthylalkylamides correspondants, à l'aide de catalyseurs du type chromite de cuivre, sous flux d'hydrogène circulant, à des pressions comprises entre 10 et 100 bars, et à des températures comprises entre 220 et 280 ° C, le catalyseur contenant de façon caractéristique, outre de l'oxyde de cuivre et du chromite de cuivre, de l'oxyde de manganèse.

On entend par catalyseur au chromite de cuivre dopé des compositions essentiellement à base d'oxydes de cuivre et de chrome, contenant quelques pourcents d'un oxyde d'un autre métal, tel le baryum, le magnésium ou le calcium, dont le rôle est de stabiliser le catalyseur en retardant la réduction du cuivre quand le catalyseur fonctionne en hydrogénation, moyennant une certaine perte d'activité et de sélectivité. Les catalyseurs au chromite de cuivre dopés au baryum sont utilisés pour catalyser l'hydrogénation des aldéhydes en alcools. Il n'était pas connu que des chromites de cuivre dopés au manganèse soient avantageux dans l'hydrogénation des amides en amines pratiquement exemptes d'alcools. C'est ce comportement inattendu d'un tel catalyseur que l'on met à profit dans l'invention pour produire les N,N-diméthyl-N-alkylamines de qualité souhaitée.

Pour préparer le catalyseur utile pour l'invention, on peut opérer :

- soit en coprécipitant par une base les oxydes de cuivre, de chrome et de manganèse à partir d'une solution de leurs sels;
- soit en coprécipitant par une base les oxydes de cuivre et de manganèse sur l'oxyde chromique;
- soit en décomposant thermiquement un sel mixte de cuivre et de chrome en présence d'oxyde ou d'un sel de manganèse.

Le produit de décomposition thermique, ou le coprécipité, une fois lavé et séché à l'étuve, est calciné sous air à une température supérieure à 350 ° C et n'excédant pas 600 ° C, et de préférence à une température de l'ordre de 450 ° C, puis réduit de préférence par l'hydrogène, à une température comprise entre 100 et 300 ° C.

Les compositions catalytiques selon l'invention contiennent de 10 à 75% en poids d'oxyde de cuivre CuO, de 10 à 75% d'oxyde de chrome Cr2O3 et de 2 à 20% d'oxyde de manganèse MnO2. On a préféré des compositions de 45 à 49% de CuO, 46 à 49% de Cr2O3 et 2 à 10% de MnO2. Le catalyseur se présente le plus souvent sous forme pulvérulente noire; il peut être présenté en tablettes ou en granulés extrudés. Certaines de ces compositions sont d'ailleurs commerciales.

En ce qui concerne la conduite générale du procédé discontinu de fabrication de N,N-diméthyl-N-alkylamines selon l'invention, les indications qui suivent consituent le meilleur mode de réalisation del'invention.

On utilise des réacteurs équipés d'une turbine et de pales d'agitation, construits de façon à pouvoir fonctionner à une presion de l'ordre de 100 bars, que l'on charge avec le N,N-diméthylalkylamide et de 0,5 à 20% en poids du catalyseur, les quantités d'environ 5% étant celles qui seront retenues dans la pratique.

Il y a nécessité d'évacuer l'eau formée par la réaction

$$R-CO-N<\begin{matrix}CH_3\\CH_3\end{matrix} + 2\ H_2 \longrightarrow R-CH_2-N<\begin{matrix}CH_3\\CH_3\end{matrix} + H_2O$$

pour permettre son avancement et pour éviter le blocage du catalyseur. On utilise pour cela la circulation de l'hydrogène sous pression ; l'eau est condensée et l'hydrogène est recyclé à l'aide d'un surpresseur.

On fait évoluer la réaction à des températures comprises entre 180 et 300 ° C, et préférentiellement entre 220 et 280 ° C, et sous une pression totale comprise entre 10 et 100 bars. La réaction procède plus rapidement sous pression élevée, mais au détriment de la sélectivité, notamment du fait d'une plus grande contribution de la production d'alcools selon la réaction

$$R{-}CO{-}N{<}\begin{matrix}CH_3\\ CH_3\end{matrix} \quad + \quad 2\ H_2 \longrightarrow R{-}CH_2OH \quad + \quad HN{<}\begin{matrix}CH_3\\ CH_3\end{matrix}$$

De ce fait, on préfère opérer entre 10 et 50 bars.

Le flux d'hydrogène circulant est compris entre 0,2 litre d'$H_2$/g d'amide/heure et 2 litres d'$H_2$/g d'amide/heure (soit très sensiblement entre 0,2 mole $H_2$/mole amide/heure et 2 moles $H_2$/mole amide/heure). Le choix de débits sensiblement plus élevés n'est suivi d'aucune amélioration de l'activité ni de la sélectivité du catalyseur.

Bien que le choix du catalyseur dopé au manganèse et des conditions opératoires indiquées conduisent à un faible taux d'alcools dans les produits de réaction, il est nécessaire de transformer en amines les alcools qui ont pu néanmoins se former. Ce que l'on réalise par addition de diméthylamine, grace à la réaction

$$R{-}CH_2{-}OH \quad + \quad H{-}N{<}\begin{matrix}CH_3\\ CH_3\end{matrix} \longrightarrow R{-}CH_2{-}N{<}\begin{matrix}CH_3\\ CH_3\end{matrix} \quad + \quad H_2O$$

La proportion de diméthylamine n'excède généralement pas 1% molaire (22,5% en poids) de l'hydrogène circulant. Il y a intérêt à n'introduire la diméthylamine que vers la fin de la réaction, lorsque l'alcalinité a atteint environ 75% de l'alcalinité théorique.

Compte tenu des limitations diffusionnelles des espèces chimiques engagées dans la réaction, et notamment de la relativement faible diffusion de l'hydrogène dans l'amide en l'absence de solvant, il faut maintenir dans le milieu une agitation vigoureuse. Mais bien que la cinétique globale soit très sensible à la vitesse d'agitation, au delà d'une certaine limite on n'observe plus d'accroissement sensible de la vitesse de la réaction. Cette limite est évidemment fonction de l'appareillage utilisé. C'est cette limite expérimentale qui figure dans les exemples donnés ci-après à titre d'illustration.

L'invention s'étend sans difficulté à l'obtention de N,N-diméthyl-N-alkylamines saturées autres que les alkyl($C_{12}$-$C_{14}$)-amines, notamment aux N,N-diméthyl-N-alkylamines qui trouvent leur origine dans les acides gras d'origine animale ou végétale, par exemple les acides gras de suif (en moyenne $C_{16}$-$C_{18}$), de soja (en moyenne $C_{16}$-$C_{18}$ insaturés) ou de colza (en moyenne $C_{18}$-$C_{22}$), et se transpose à l'obtention d'homologues tels que les N,N-diéthyl-N-alkylamines à partir des N,N-diéthylalkylamides. Son adaptation à un procédé continu de production de N,N-diméthyl-N-alkylamines est également à la portée de l'homme du métier.

## EXEMPLES

<u>Exemple 1</u> : Préparation d'un catalyseur Cu-Cr-Mn

On prépare un catalyseur massique Cu-Cr-Mn en mélangeant dans 500 ml d'eau distillée 89 grammes de $Cu(NO_3)_2$, $6H_2O$, 240 grammes de $Cr(NO_3)_3$, 9 $H_2O$ et 10 grammes de $Mn(NO_3)$, $4H_2O$. On précipite les hydroxydes sous agitation en additionnant une solution 2 M de $Na_2CO_3$ (212 grammes pour 1 litre d'eau) jusqu'à un pH de 6,5. On filtre le précipité, on lave abondamment à l'eau distillée et l'on sèche la pâte obtenue à 150 °C à l'étuve pendant 16 heures. Aprés séchage le solide est broyé en fine poudre (2 à 10 µm) et à nouveau calciné sous air dans un four à une température de 450 °C. La poudre initialement verte vire au noir.

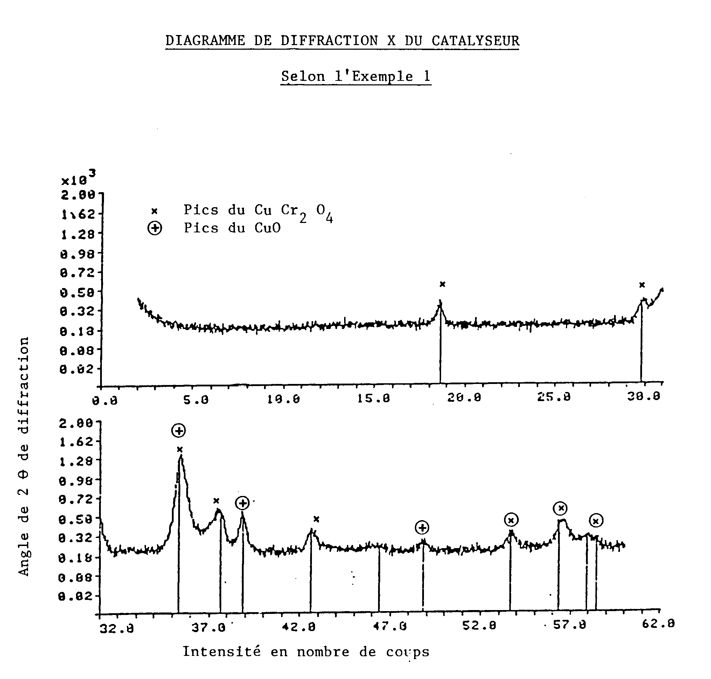

On obtient ainsi un catalyseur dont la composition réelle s'établit à CuO = 46% , $Cr_2O_3$ = 46% , $MnO_2$ = 4% , les pourcentages étant exprimés en poids. Ce produit est très partiellement cristallisé. On en a donné sur la planche 1/1 le diagramme de diffraction X, sur lequel on distingue les pics caractéristiques du chromite $CuCr_2O_4$, et ceux de l'oxyde de cuivre CuO. Les pics de l'oxyde de manganèse en sont absents, ce qui témoigne de la présence de l'oxyde de manganèse dans la phase amorphe.

Exemple 2

On met en oeuvre le catalyseur de l'exemple 1 dans l'hydrogénation d'amides N,N-diméthylés de longueur de chaîne carbonée essentiellement $C_{12}$-$C_{14}$ (0,15 % de $C_8$, 0,2 % de $C_{10}$, 66 % de $C_{12}$, 33 % de $C_{14}$).

De tels N,N-diméthylalkylamides s'obtiennent industriellement par amidation directe des acides gras correspondants par la diméthylamine, par exemple en maintenant l'acide gras à 170-190°C pendant 10 à 16 heures sous circulation de diméthylamine à 0,2-2 bars, puis en éliminant sous vide l'excès de diméthylamine piègée dans le N,N-diméthylalkylamide formé. Dans le cas du présent exemple, le diméthylamide utilisé a une alcalinité amide égale à 4,2 moles/kg et contient 0,44 % d'acide gras $C_{12}$-$C_{14}$ résiduel et 0,02 % d'eau.

On charge 400 g de ce N,N-diméthyl($C_{12}$-$C_{14}$)amide et 20 g du catalyseur Cu-Cr-Mn dans un autoclave d'un litre. Après purge du réacteur à l'azote, sous une agitation par pales de 2000 t/min. On monte à une pression de 27 bars d'hydrogène pur et l'on établit une circulation de 400 l/h d'hydrogène. On monte en température jusqu'à 235° C à raison de 5° C/min et l'on remplace alors l'hydrogène pur par un mélange $H_2$-diméthylamine à 0,8 % de diméthylamine dans 400 l/h d'$H_2$. On prélève des échantillons de façon à suivre la cinétique de la réaction par mesure de l'alcalinité amide ou par analyse chromatographique. On arrête la réaction en coupant l'alimentation en température et l'agitation, et en ramenant la pression à la pression atmosphérique, un quart d'heure après l'obtention d'une alcalinité amide nulle.

Le catalyseur décante alors à chaud dans la N,N-diméthyl($C_{12}$-$C_{14}$)amine formée, et l'on prélève celle-ci par aspiration, après décompression de l'autoclave par un orifice situé sur le chapeau du réacteur ; la pâte catalytique reste au fond du réacteur.

Pour les opérations suivantes, on recharge le réacteur avec 400 grammes d'amide et 4 grammes de catalyseur Cu-Cr-Mn (pour compenser notamment les pertes dues à la prise d'échantillons). Les résultats obtenus dans la première opération sur catalyseur neuf, et dans les cinq opérations suivantes sur catalyseur recyclé, sont consignés dans le tableau 1.

On constate d'une part que le temps total de réaction évolue peu d'un recyclage à l'autre, ce qui témoigne de la stabilité catalytique, et que la teneur en alcool des produits finis est maintenue inférieure à 0,5 % . Le sous-produit principal de la réaction est la dialkylméthylamine facilement séparable de l'alkyldiméthylamine par distillation. On obtient aussi des esters à chaîne grasse en proportion inférieure à 1 % . Le rendement moyen en alkyldiméthylamine est de 87,3 % en poids.

Tableau 1

HYDROGENATION DU DIMETHYLALKYLAMIDE C12 C14

CONDITIONS OPERATOIRES

Catalyseur Cu/Cr/Mn : 20 g
Charge : 400 g
Débit Gazeux du mélange $H_2$-DMA à 0,8 % : 400 l/h

Pression : 27 bars
Température : 235° C
Agitation : 2000 tr/min

| No Recyclage | Temps de Réaction | Conversion Amide | Alcalinité Amine (mole / kg) | COMPOSITION % POIDS | | | | |
|---|---|---|---|---|---|---|---|---|
| | | | | R - OH | $R-N(CH_3)_2$ | $(R)(R')N-CH_3$ | $R^{-1}-C(=O)-N(CH_3)_2$ | Esters lourds |
| Catalyseur neuf | 7 h 00 | 99.9% | 4.2 | 0.3 | 88.5 | 10.5 | 0.1 | 0.6 |
| 1er | 7 h 45 | 99.8% | 4.2 | 0.5 | 86.7 | 12.5 | 0.2 | 0.1 |
| 2ème | 9 h 00 | 100.0% | 4.2 | 0.0 | 88 | 11.9 | 0.0 | 0.1 |
| 3ème | 9 h 00 | 99.4% | 4.2 | 0.4 | 87.7 | 10.4 | 0.6 | 0.9 |
| 4ème | 9 h 00 | 99.8% | 4.3 | 0.3 | 89.3 | 9.8 | 0.2 | 0.4 |
| 5ème | 9 h 30 | 99.7% | 4.3 | 0.2 | 84.1 | 14.8 | 0.3 | 0.6 |

Dans le présent tableau R a la signification suivante : Chaîne hydrocarbonée C12 - C14 dont 0,15 % de C8 - 0,2 % de C10 - 66 % C12 - 33 % C14).
$R^{-1}C(=O)-$ représente des chaînes à même répartiton de carbones.

Exemple 3

A titre de comparaison on a appliqué les mêmes conditions opératoires que dans l'exemple 2, notamment en pratiquant le même type de recyclage, mais avec un catalyseur Cu-Cr-Ba contenant 40 %

de CuO, 45,5 % de $Cr_2O_3$ et 9,5 % de BaO, dont la surface spécifique est de 36 $m^2$/g. Ce catalyseur a été préparé comme dans l'exemple, à la différence d'emploi de $Ba(NO_3)_2$, $H_2O$ au lieu et place de $Mn(NO_3)$, $4H_2O$.

Les résultats obtenus dans la première opération sur catalyseur neuf et dans les trois opérations suivantes sur catalyseur recyclé, sont consignés dans le tableau 2.

On observe ici une augmentation du temps de réaction nécessaire à la conversion de l'amide N,N-diméthylé, due à la désactivation du catalyseur. De plus on observe que la teneur en alcool ne peut être amenée à une teneur inférieure à 3 % , ce qui est rédhibitoire. La poursuite de la réaction au-delà du temps nécessaire à la conversion totale ne parvient pas à réduire ce taux d'alcool.

Tableau 2

HYDROGENATION DU DIMETHYLALKYLAMIDE C12 C14

CONDITIONS OPERATOIRES

Catalyseur Cu-Cr-Ba : 20 g
Charge : 400 g
Débit Gazeux du mélange $H_2$-DMA à 0,8 % : 400 l/h

Pression : 27 bars
Température : 235° C
Agitation : 2000 tr/min

| No Recyclage | Temps de Réaction | Conversion Amide | Alcalinité Amine (mole / kg) | COMPOSITION % POIDS | | | | |
|---|---|---|---|---|---|---|---|---|
| | | | | R - OH | 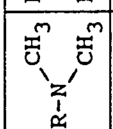 | 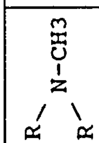 | 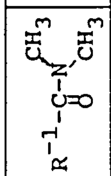 | Autres lourds |
| Catalyseur neuf | 7 h 00 | 99.5% | 4.2 | 3.5 | 88.3 | 7 | 0.6 | 0.6 |
| 1er | 7 h 45 | 99.3% | 4.1 | 7.1 | 85.5 | 5.7 | 0.7 | 1.0 |
| 2ème | 9 h 00 | 97.2% | 4.2 | 5.0 | 84.8 | 7 | 2.8 | 0.4 |
| 3ème | 11 h 00 | 99.5% | 4.0 | 4.1 | 86.1 | 8.5 | 0.5 | 0.8 |
| 4ème | 10 h 45 | 96.7% | 3.8 | 8.6 | 80.7 | 6.4 | 3.3 | 1.0 |

Pour la signification de R et $R^{-1}$-C-, voir tableau 1
‖

Exemple 4

On utilise dans cet exemple comparatif un catalyseur au chromite de cuivre non dopé résultant de la préparation selon l'exemple 1 sans $Mn(NO_3)$, $4H_2O$.

Les résultats des tests catalytiques au cours de deux recyclages sont dans le tableau 3.

On remarque que le catalyseur est très actif à la première utilisation mais que la teneur en alcool des produits finis est très forte (> 10 %), et que le temps de réaction augmente très sensiblement au cours des recyclages ultérieurs sans que la teneur en alcool puisse être maîtrisée.

Tableau 3

HYDROGENATION DU DIMETHYLALKYLAMIDE $C_{12}$—$C_{14}$

Catalyseur Cu-Cr : 20 g
Charge : 400 g d'amide
Débit gazeux du mélange $H_2$-0,8 % DMA : 400 l/g

Pression : variable
Température : variable
Agitation : 2000 tr/min

| No Recyclage | Temps de Réaction | Conversion Amide | Pression (bars) | Temp. (°C) | Alcalinité Amine (mole / kg) | COMPOSITION % POIDS R - OH | $R-N(CH_3)_2$ | $R_2N-CH_3$ | $R^{-1}-C(O)-N(CH_3)_2$ | Autres lourds |
|---|---|---|---|---|---|---|---|---|---|---|
| Catalyseur neuf | 6 h 00 | 100.0% | 50 | 235 | 3.8 | 10.9 | 80.7 | 7.2 | 0.2 | 1.0 |
| 1er | 9 h 15 | 99.6% | 27 | 240 | 4.1 | 4.9 | 86.7 | 7.0 | 0.5 | 0.9 |
| 2ème | 10 h 00 | 98.7% | 27 | 240 | 3.9 | 6.7 | 87.5 | 3.9 | 1.0 | 0.9 |

Pour la signification de R et $R^{-1}$-C , voir tableau 1.

Exemple 5

Dans un réacteur semi-industriel, on a chargé 100 kg de N,N-diméthylalkylamide obtenu à partir d'un acide gras industriel $C_{12-14}$ avec une répartition de chaîne grasse :

| | |
|---|---|
| acides en $C_{12}$ | 70% |
| acides en $C_{14}$ | 30% |

et 5 kg de catalyseur selon l'invention dont la composition est 47 % de CuO, 49 % de $Cr_2O_3$, 4 % de $MnO_2$. Après purge du réacteur à l'azote, on admet de l'hydrogène pur pour monter la pression totale vers 30 bars, et on établit une circulation de 100 m3 P.T.N./h d'hydrogène. La température est élevée progressivement à 235 °C, et on maintient ces conditions durant 5 heures. Lorsque des prises d'essais présentent une alcalinité égale ou supérieures à 3,3 milliéquivalents/gramme, on introduit dans le flux circulant de la diméthylamine à raison de 1 mole % mole d'hydrogène, et on poursuit la réaction pendant encore deux heures. Après arrêt de la circulation et décompression, on vidange le récteur, on laisse décanter le catalyseur, et on prélève le surnageant.
On obtient ainsi une amine brute qui titre 93% de diméthyl($C_{12}$-$C_{14}$) amine.

Les opérations suivantes suivent le même schéma après rechargement de 100 kg de N,N-diméthylalkylamide mélangés à la pâte catalytique récupérée de l'opération précédente, et de 0,5 kg de catalyseur neuf. La 10 ème opération a délivré 87 kg d'amine brute dont la composition était :

| | |
|---|---|
| N,N-diméthyl($C_{12}$-$C_{14}$) amine | 92% |
| N-méthyl-N,N-di($C_{12}$-$C_{14}$) amine | 7% |
| alcool ($C_{12}$-$C_{14}$) | 0,2% |
| autres | 0,8% |

A noter que le rendement en amine brute de 92% n'est qu'un rendement apparent. En fait, une partie non négligeable de produits gras (essentiellement amines avec un peu d'amides) est entrainée par le flux d'hydrogène, circulant en même temps que l'eau éliminée dans la réaction. Dans une opération industrielle, ces produits dits entraînements gras, sont séparés et recyclés dans l'opération suivante. Dans le présent exemple, les entrainements gras recyclables sont de 5 kg.

Exemple 6

On a procédé comme dans l'exemple 5, à la différence que l'on a utilisé un acide gras de coprah de composition :

| | |
|---|---|
| acides en $C_8$ | 5,5% |
| acides en $C_{10}$ | 6,3% |
| acides en $C_{12}$ | 50,9% |
| acides en $C_{14}$ | 18,3% |
| acides en $C_{16}$ | 8,6% |
| acides en $C_{18}$ | 9,7% |

La dixième opération a délivré 84 kg d'amines brutes et 8 kg d'entraînements gras recyclables.

Exemple 7

On a procédé comme dans l'exemple 5, à la différence que la diméthylamine est introduite dès que l'on a atteint la température de fonctionnement (de 235 °C), à un moment où l'alcalinité développée est encore très faible. L'amine brute obtenue au premier cycle ne titre plus que 90 % d'amines tertiaires du type diméthyl-($C_{12}$-$C_{14}$) amine, ce qui démontre l'intérêt de la pratique, selon l'invention, de n'introduire la diméthylamine que lorsque l'alcalinité s'est établie à environ 75% de la valeur théorique.

## Revendications

**1.** Procédé pour l'obtention de N,N-diméthyl-N-alkylamines de formule générale

$$R - N < \begin{matrix} CH_3 \\ CH_3 \end{matrix}$$

où R est un reste alkyle ou alkényle comprenant de 12 à 24 atomes de carbone, lesdites N,N-diméthyl-N-alkylamines contenant moins de 1% d'alcanols, par hydrogénation catalytique sur catalyseurs du type chromite de cuivre, de N,N-diméthylalkylamides, sous flux d'hydrogène circulant dont le rapport molaire au N,N-diméthylalkylamide est compris entre 2 et20, la pression totale étant comprise entre 10 et 100 bars, la température étant comprise entre 220 et 280 °C, caractérisé en ce que le catalyseur contient, outre l'oxyde de cuivre et du chromite de cuivre, de l'oxyde de manganèse.

**2.** Procédé pour l'obtention de N,N-diméthyl-N-alkylamines selon la revendication 1, caractérisé en ce que le reste alkyle ou alkényle comprend surtout des chaînes à 12-14 atomes de carbone, et que le N,N-diméthylalkylamide utilisé est un N,N-diméthylamide d'acides de coprah.

**3.** Procédé pour l'obtention de N,N-diméthyl-N-alkylamines selon les revendications 1 ou 2, caractérisé en ce que la répartition pondérale du catalyseur en oxydes est :

| | |
|---|---|
| CuO | 10-75% |
| $Cr_2O_3$ | 10-75% |
| $MnO_2$ | 2-20% |

**4.** Procédé pour l'obtention de N,N-diméthyl-N-alkylamines selon les revendications 1 ou 2, caractérisé en ce que la répartition pondérale du catalyseur en oxydes est :

| | |
|---|---|
| CuO | 45-49% |
| $Cr_2O_3$ | 45-49% |
| $MnO_2$ | 2-10% |

**5.** Procédé pour l'obtention de N,N-diméthyl-N-alkylamines selon les revendications 1 à 4, caractérisé en ce que le catalyseur est utilisé en proportion pondérale par rapport au N,N-diméthylalkylamide comprise entre 2 et 10% .

**6.** Procédé pour l'obtention de N,N-diméthyl-N-alkylamines selon les revendications 1 à 5, caractérisé en ce que le flux d'hydrogène circulant contient environ 1% en volume de diméthylamine.

**7.** Procédé pour l'obtention de N,N-diméthyl-N-alkylamines selon la revendication 6, caractérisé en ce que la diméthylamine est introduite dans le flux circulant lorsque l'alcalinité a atteint 75% de la valeur théorique attendue.

## Claims

**1.** Process for obtaining N,N-dimethyl-N-alkylamines of general formula

$$R - N < \begin{matrix} CH_3 \\ CH_3 \end{matrix}$$

where R is an alkyl or alkenyl residue comprising from 12 to 24 carbon atoms,
the said N,N-dimethyl-N-alkylamines containing less than 1% of alkanols, by catalytic hydrogenation, on catalysts of the copper chromite type, of N,N-dimethylalkylamides, under a circulating flow of hydrogen, the mole ratio of which to the N,N-dimethylalkylamide is between 2 and 20, the total pressure being between 10 and 100 bars, the temperature being between 220 and 280 ° C,
characterised in that the catalyst contains, apart from copper oxide and copper chromite, manganese dioxide.

2. Process for obtaining N,N-dimethyl-N-alkylamines according to Claim 1, characterised in that the alkyl or alkenyl residue most especially comprises chains containing 12-14 carbon atoms, and in that the N,N-dimethylalkylamide used is an N,N-dimethylamide of coconut acids.

3. Process for obtaining N,N-dimethyl-N-alkylamines according to Claim 1 or 2, characterised in that the weight distribution of the catalyst with respect to oxides is:

| | |
|---|---|
| CuO | 10-75% |
| $Cr_2O_3$ | 10-75% |
| $MnO_2$ | 2-20% |

4. Process for obtaining N,N-dimethyl-N-alkylamines according to Claim 1 or 2, characterised in that the weight distribution of the catalyst with respect to oxides is:

| | |
|---|---|
| CuO | 45-49% |
| $Cr_2O_3$ | 45-49% |
| $MNO_2$ | 2-10% |

5. Process for obtaining N,N-dimethyl-N-alkylamines according to Claims 1 to 4, characterised in that the catalyst is used in a weight proportion relative to the N,N-dimethylalkylamide of between 2 and 10%.

6. Process for obtaining N,N-dimethyl-N-alkylamines according to Clams 1 to 5, characterised in that the circulating flow of hydrogen contains approximately 1% by volume of dimethylamine.

7. Process for obtaining N,N-dimethyl-N-alkylamines according to Claim 6, characterised in that the dimethylamine is introduced into the circulating flow when the alkalinity has reached 75% of the expected theoretical value.

## Patentansprüche

1. Verfahren zur Herstellung von N,N-Dimethyl-N-alkylaminen der allgemeinen Formel

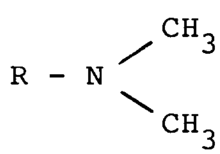

in der R ein Alkyl- oder Alkylenrest mit 12 bis 24 Kohlenstoffatomen ist,
wobei die N,N-Dimethyl-N-alkylamine weniger als 1% Alkanole enthalten, durch katalytische Hydrierung von N,N-Dimethylalkylamiden mit Katalysatoren vom Kupferchromittyp, unter einem zirkulierendem Strom von Wasserstoff, dessen Molverhältnis zum N,N-Dimethylalkylamid zwischen 2 und 20 liegt, bei einem Gesamtdruck zwischen 10 und 100 bar und einer Temperatur zwischen 220 und 280 ° C,
dadurch gekennzeichnet, daß der Katalysator außer Kupferoxid und Kupferchromit Manganoxid enthält.

2. Verfahren zur Herstellung von N,N-Dimethyl-N-alkylaminen nach Anspruch 1, dadurch gekennzeichnet, daß der Alkyl- oder Alkenylrest vor allem Ketten mit 12 bis 14 Kohlenstoffatomen enthält und daß das verwendete N,N-Dimethylalkylamid ein N,N-Dimethylamid aus Koprasäuren ist.

3. Verfahren zur Herstellung von N,N-Dimethyl-N-alkylaminen nach einem der Ansprüche 1 oder 2, dadurch gekennzeichnet, daß die Gewichtsverteilung der Oxide im Katalysator ist:

| | |
|---|---|
| $CuO$ | 10-75% |
| $Cr_2O_3$ | 10-75% |
| $MnO_2$ | 2-20% |

4. Verfahren zur Herstellung von N,N-Dimethyl-N-alkylaminen nach einem der Ansprüche 1 oder 2, dadurch gekennzeichnet, daß die Gewichtsverteilung der Oxide im Katalysator ist:

| | |
|---|---|
| $CuO$ | 45-49% |
| $Cr_2O_3$ | 45-49% |
| $MnO_2$ | 2-10% |

5. Verfahren zur Herstellung von N,N-Dimethyl-N-alkylaminen nach den Ansprüchen 1 bis 4, dadurch gekennzeichnet, daß der Katalysator, bezogen auf das N,N-Dimethyl-N-alkylamid, in einem Gewichtsverhältnis zwischen 2 und 10% verwendet wird.

6. Verfahren zur Herstellung von N,N-Dimethyl-N-alkylaminen nach den Ansprüchen 1 bis 5, dadurch gekennzeichnet, daß der zirkulierende Wasserstoffstrom etwa 1 Volumen% Dimethylamin enthält.

7. Verfahren zur Herstellung von N,N-Dimethyl-N-alkylaminen nach Anspruch 6, dadurch gekennzeichnet, daß das Dimethylamin dem zirkulierenden Strom zugefügt wird, wenn die Alkalinität 75% des theoretisch erwarteten Wertes erreicht hat.

DIAGRAMME DE DIFFRACTION X DU CATALYSEUR

Selon l'Exemple 1